# DEMANDE DE BREVET EUROPEEN

(11) **EP 4 368 723 A1**
(43) Date de publication de la demande: **15.05.2024**
(21) Numéro de dépôt: 22206665.6
(22) Date de dépôt: 10.11.2022
(51) Int. Cl.: C12Q 1/04, C12Q 1/06, C12Q 1/08

(54) **MILIEU DE CULTURE POUR DÉTECTER DES MICROORGANISMES COMPRENANT UN MÉLANGE D'AGAR ET DE KAPPA-CARRAGHÉNANE EN TANT QU'AGENT GÉLIFIANT**

(71) Demandeur: bioMérieux, 69280 Marcy l'Etoile (FR)
(72) Inventeur: MAO, Bosi, 69007 Lyon (FR); LEBLANC, Laurent, 69730 Genay (FR); SCHNEIDER, Vivian, 69380 Chazay d'Azergues (FR)
(74) Mandataire: bioMérieux PI Groupement mandataires

(57) **Abrégé**

La présente invention concerne le domaine de la microbiologie et plus particulièrement celui de la microbiologie industrielle et clinique. De manière plus spécifique, la présente invention concerne un milieu de culture pour détecter des microorganismes comprenant, en tant qu'agent gélifiant, un mélange d'agar et de κ-carraghénane.

## Description

### DOMAINE TECHNIQUE

La présente invention concerne le domaine de la microbiologie et plus particulièrement celui de la microbiologie industrielle et clinique. De manière plus spécifique, la présente invention concerne un nouveau milieu de culture pour détecter des microorganismes comprenant un mélange d'agar et de κ-carraghénane en tant qu'agent gélifiant.

### TECHNIQUE ANTERIEURE

La détection et l'identification de microorganismes est très importante dans les domaines cliniques et industriels. Dans les industries pharmaceutiques et agro-alimentaires, les milieux de culture sont toujours la référence pour détecter et dénombrer des micro-organismes. L'industrie agroalimentaire utilise ces produits pour les contrôles microbiologiques des produits destinés à l'alimentation humaine et animale. Du côté de l'industrie pharmaceutique, les milieux sont utilisés pour effectuer principalement des contrôles de l'environnement (air, surface, opérateur, etc.) et pour vérifier qu'il n'y a pas de contamination bactérienne dans les produits en cours de fabrication. Bien souvent les contrôles effectués entrent en considération pour la libération des lots de produits finis ou semi-finis. De nombreux essais et modes opératoires dépendent de l'aptitude des milieux de culture à donner des résultats homogènes et reproductibles. Les exigences relatives aux milieux peuvent être spécifiques à la fois de l'échantillon et des souches à rechercher. Des milieux de culture satisfaisant à des critères de performance établis, constituent donc un préalable à toute analyse microbiologique fiable. Les milieux prêts à l'emploi sont fabriqués dans des conditions de production standardisées et résolvent de nombreux problèmes dans les laboratoires de microbiologie où le temps, l'équipement ou le personnel formé font souvent défaut. Ainsi, les milieux de culture prêts à l'emploi, de qualité reconnue, permettent une analyse microbiologique fiable.

Ils peuvent être sous forme de milieu liquide, solide ou semi-solide fournis dans des boîtes, flacons, tubes ou autres contenants.

Un composé majeur des milieux de culture solide ou semi-solide est l'agent gélifiant et notamment l'agar. Ce dernier est extrait d'algues rouges de la famille des Rhodophytes. L'agar présente l'inconvénient d'être issu d'une ressource naturelle limitée. Ainsi, Lines (Applied and Environmental Microbiology, Dec 1977, p 637-639) décrit l'utilisation de κ-carraghénanes comme substituant de l'agar dans un milieu de culture. L'inconvénient d'un milieu de culture dont l'agar a été substitué par du κ-carraghénane est qu'il commence à se solidifier à des températures élevées. Alors que l'agar est dispensé à une température approximative de 45-55°C, un milieu de culture à base de κ-carraghénanes nécessite, pour être dispensé dans les boites, d'être à une température supérieure à 60°C. Cela a pour conséquence des problèmes d'industrialisation de milieu de culture gélosé avec le maintien d'une température élevée tout au long du procédé de fabrication ou des risques de brûlures des techniciens si les boites de Petri sont coulées sur le lieu de leur utilisation. De plus, une température de gélification élevée n'est pas compatible avec la production de milieux de culture contenant des composés thermosensibles tels que des antibiotiques ou du sang.

D'autres part, l'agent gélifiant est également le composé clé pour une amélioration de la durée de vie des milieux de cultures. En effet, les milieux de culture à base d'agar, bien que largement utilisés, présentent des inconvénients comme par exemple la synérèse (sortie de l'eau du réseau du gel) et la rétraction qu'il est souhaitable d'éviter. Ainsi, le document WO 2004050675 décrit l'utilisation d'un mélange d'agar et de iota-carraghénane dans un milieu de culture gélosé. Ce mélange permet de limiter la synérèse du gel, améliorant ainsi sa stabilité et sa durée de vie.

Il existe néanmoins toujours un besoin de mettre au point un milieu de culture gélosé offrant des paramètres physico-chimiques et une résistance à la rétraction permettant d'améliorer sa durée de vie tout en gardant une qualité optimale et en restant compatible à une industrialisation.

### RESUME DE L'INVENTION

Un objectif de la présente invention est de proposer un milieu de culture résistant à la déshydratation par sa capacité à ne pas, ou très faiblement, se rétracter.

Un autre objectif de l'invention est de proposer un milieu de culture ayant une température de gélification compatible avec l'industrialisation ou l'utilisation de composés thermosensibles.

Ces objectifs parmi d'autres sont atteints par la présente invention qui concerne un milieu de culture pour la détection de microorganismes comprenant
- de l'agar ou de l'agarose
- du κ-carraghénane.

De façon tout à fait avantageuse, le ratio pondéral d'agar : κ-carraghénane dans le milieu de culture est compris entre 80 : 20 et 30 : 70.

Dans un mode particulier de l'invention, lorsque la concentration en sels présents dans le milieu est inférieure à 5 g/l, le ratio pondéral d'agar : κ-carraghénane est compris entre 80 : 20 et 60 : 40.

Dans un autre mode particulier de l'invention, lorsque la concentration en sels présents dans le milieu est comprise entre 5 g/l et 20 g/l, le ratio pondéral d'agar : κ-carraghénane est compris entre 80 : 20 et 30 : 70.

Dans un autre mode particulier de l'invention, lorsque la concentration en sel présent dans le milieu est supérieur à 20 g/l et le ratio pondéral d'agar : κ-carraghénane est compris entre 80 : 20 et 60 : 40.

De façon avantageuse, le milieu de culture comprend en outre un composé thermosensible, tel qu'un antibiotique et/ou du sang.

De façon avantageuse, le milieu de culture est gélifié dans une boite de Pétri.

Un autre objet de l'invention concerne un procédé d'obtention d'un milieu selon l'invention caractérisé en ce qu'il consiste essentiellement à:
- mettre en surfusion les composés du milieu de culture afin de les mélanger
- refroidir à une première température au-dessus de la température de gélification du milieu de culture
- remplir les boites de Pétri avec ce milieu de culture en solution
- laisser refroidir pour atteindre la température de gélification et permettre la formation d'un milieu de culture gélifié, la température de gélification étant comprise entre 30°C et 45°C.

Un autre objet de l'invention concerne un milieu de culture comprenant un mélange d'agar : κ-carraghénane ayant un ratio pondéral compris entre 80 : 20 et 30 : 70 et une température de gélification comprise entre 30°C et 45°C.

Un autre objet de l'invention concerne une méthode de culture microbiologique *in vitro,* dans laquelle des microorganismes susceptibles d'être présents dans un échantillon sont ensemencés dans ou sur un milieu de culture selon l'invention.

Un autre objet de l'invention concerne une méthode de détection d'un micro-organisme cible dans un échantillon susceptible de le contenir comprenant les étapes suivantes :
- mettre en contact ledit échantillon avec un milieu de culture gélifié selon l'invention
- incuber
- détecter la présence dudit microorganisme.

### DESCRIPTION DES FIGURES

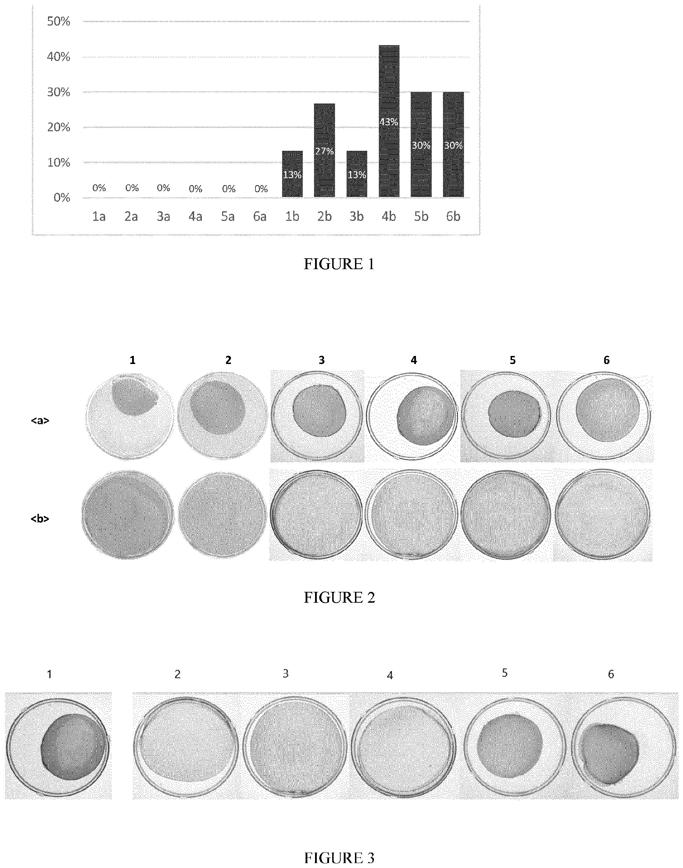

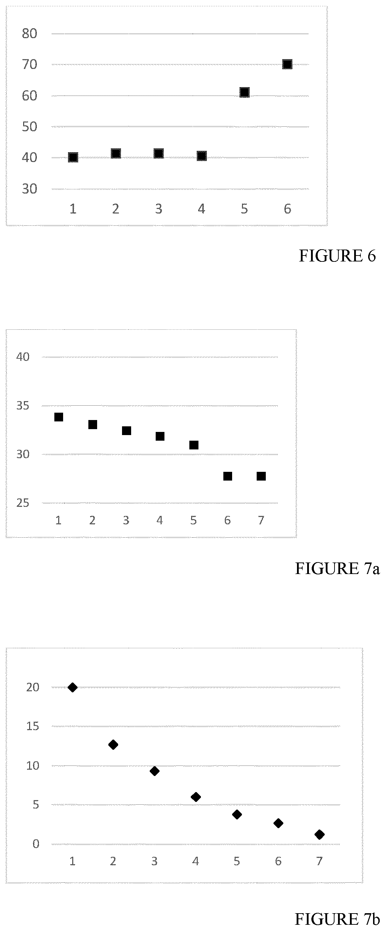

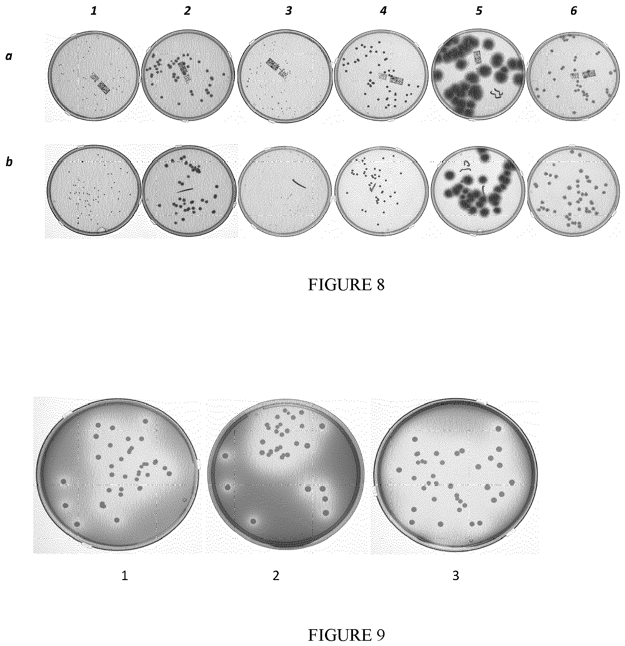

La figure 1 est un diagramme représentant le pourcentage de milieu de culture intact (non-rétracté) après le Test de Déshydratation Extrême. Les milieux de la série « a » comprennent 100% d'agar (ou d'agarose pour le milieu 1). Les milieux de la série « b » comprennent un ratio agar : κ-carraghénane de 50 : 50 (ou un ratio agarose : κ-carraghénane de 50 :50 pour le milieu 1).
   Provenance de l'agar (ou agarose pour le milieu 1) :
   Milieu la et milieu 1b : Agarose de Sigma Aldrich
   Milieu 2a et milieu 2b : Agar de Sigma Aldrich
   Milieu 3a et milieu 3b : Agar américain de Roko
   Milieu 4a et milieu 4b : Agar européen de Roko
   Milieu 5a et milieu 5b : Agar européen de Setexam
   Milieu 6a et milieu 6b : agar Gracilaria de Roko
La figure 2 représente des photos des milieux de culture après le Test de Déshydratation Extrême. Les milieux de la série « a » comprennent 100% d'agar (ou d'agarose pour le milieu 1). Les milieux de la série « b » comprennent un ratio agar : κ-carraghénane de 50 : 50 (ou un ratio agarose : κ-carraghénane de 50 :50 pour le milieu 1). Provenance de l'agar (ou agarose pour le milieu 1) :
   Milieu la et milieu 1b : Agarose de Sigma Aldrich
   Milieu 2a et milieu 2b : Agar de Sigma Aldrich
   Milieu 3a et milieu 3b : Agar américain de Roko
   Milieu 4a et milieu 4b : Agar européen de Roko
   Milieu 5a et milieu 5b : Agar européen de Setexam
   Milieu 6a et milieu 6b : agar Gracilaria de Roko
La figure 3 représente des photos des milieux de culture mix agar avec des carraghénanes différents après le Test de Déshydratation Extrême.
   Milieu 1 comprend 100% d'agar Européen (Setexam)
   Milieu 2 comprend un ratio agar : κ-carraghénane (sigma Aldrich) 50 : 50
   Milieu 3 comprend un ratio agar : κ-carraghénane (Setexam) 50 : 50
   Milieu 4 comprend un ratio agar : κ-carraghénane (Roko) 50 : 50
   Milieu 5 comprend un ratio agar : λ-carraghénane (Sigma Aldrich) 50 : 50
   Milieu 6 comprend un ratio agar : ι-carraghénane (Sigma Aldrich) 50 : 50.

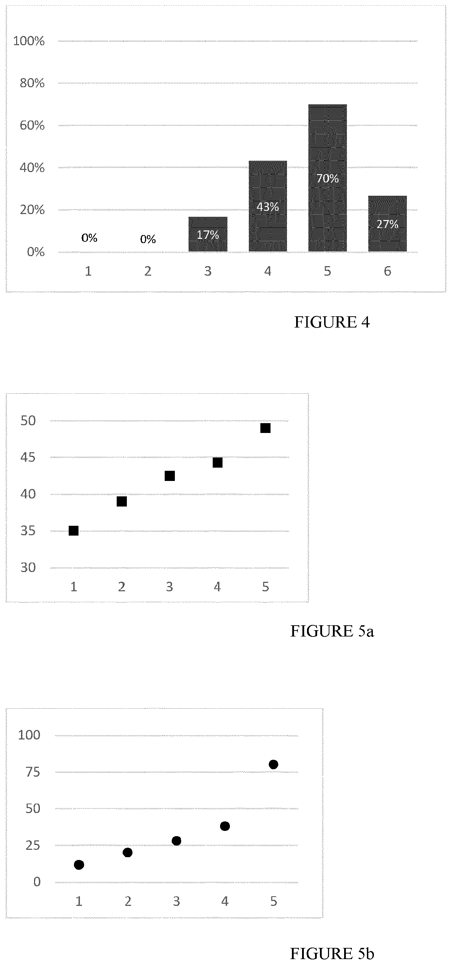

La figure 4 représente le pourcentage de milieux de cultures non rétractés pour différents ratios agar : κ-carraghénane après le test de de déshydratation extrême.
   Milieu 1 comprend 100% d'agar Européen
   Milieu 2 comprend un ratio agar : κ-carraghénane 70 : 30
   Milieu 3 comprend un ratio agar : κ-carraghénane 60 : 40
   Milieu 4 comprend un ratio agar : κ-carraghénane 50 : 50
   Milieu 5 comprend un ratio agar : κ-carraghénane 30 : 70
   Milieu 6 comprend un ratio agar : κ-carraghénane 0 : 100
La figure 5 (a) représente la température de gélification de milieux TSA modifiés avec différents ratios agar : κ-carraghénane.
   Milieu 1 comprend 100% d'agar
   Milieu 2 comprend un ratio agar : κ-carraghénane 70 : 30
   Milieu 3 comprend un ratio agar : κ-carraghénane 50 : 50
   Milieu 4 comprend un ratio agar : κ-carraghénane 30 : 70
   Milieu 5 comprend un ratio agar : κ-carraghénane 0 : 100
La figure 5 (b) représente la viscosité de milieux TSA modifiés avec différents ratios agar : κ-carraghénane.
   Milieu 1 comprend 100% d'agar
   Milieu 2 comprend un ratio agar : κ-carraghénane 70 : 30
   Milieu 3 comprend un ratio agar : κ-carraghénane 50 : 50
   Milieu 4 comprend un ratio agar : κ-carraghénane 30 : 70
   Milieu 5 comprend un ratio agar : κ-carraghénane 0 : 100
La figure 6 représente la température de gélification de milieux CHAPMAN modifiés avec différents ratios agar : κ-carraghénane.
   Milieu 1 comprend 100% d'agar
   Milieu 2 comprend un ratio agar : κ-carraghénane 80 : 20
   Milieu 3 comprend un ratio agar : κ-carraghénane 70 : 30
   Milieu 4 comprend un ratio agar : κ-carraghénane 60 : 40
   Milieu 5 comprend un ratio agar : κ-carraghénane 50 : 50
   Milieu 6 comprend un ratio agar : κ-carraghénane 0 : 100
La figure 7(a) représente la température de gélification de milieux SDA modifiés avec différents ratios agar : κ-carraghénane
   Milieu 1 comprend 100% d'agar
   Milieu 2 comprend un ratio agar : κ-carraghénane 80 : 20
   Milieu 3 comprend un ratio agar : κ-carraghénane 70 : 30
   Milieu 4 comprend un ratio agar : κ-carraghénane 60 : 40
   Milieu 5 comprend un ratio agar : κ-carraghénane 50 : 50
   Milieu 6 comprend un ratio agar : κ-carraghénane 30 : 70
   Milieu 7 comprend un ratio agar : κ-carraghénane 0 : 100
La figure 7 (b) représente le module élastique de milieux SDA modifiés avec différents ratios agar : κ-carraghénane
   Milieu 1 comprend 100% d'agar
   Milieu 2 comprend un ratio agar : κ-carraghénane 80 : 20
   Milieu 3 comprend un ratio agar : κ-carraghénane 70 : 30
   Milieu 4 comprend un ratio agar : κ-carraghénane 60 : 40
   Milieu 5 comprend un ratio agar : κ-carraghénane 50 : 50
   Milieu 6 comprend un ratio agar : κ-carraghénane 30 : 70
   Milieu 7 comprend un ratio agar : κ-carraghénane 0 : 100
La figure 8 correspond à des photos après incubation des milieux de culture inoculés par différents microorganismes.
   La ligne (a) correspond à un milieu TSA comprenant 100% agar et la ligne (b) correspond à un milieu TSA modifié comprenant un ratio agar : κ-carraghénanes 70 : 30
   Colonne 1 correspond à un ensemencement de *S. aureus*
   Colonne 2 correspond à un ensemencement de *B. subtilis*
   Colonne 3 correspond à un ensemencement de *P. aeruginosa*
   Colonne 4 correspond à un ensemencement de C. *albicans*
   Colonne 5 correspond à un ensemencement de *A. brasiliensis*
   Colonne 6 correspond à un ensemencement de *E.coli*
La figure 9 correspond à des photos des milieux de culture CHAPMAN modifiés après incubation inoculés avec *Staphylococcus aureus.*
   Le milieu 1 correspond à un milieu ayant 100 % d'agar.
   Le milieu 2 comprend à un ratio agar : κ-carraghénanes 80: 20.
   Le milieu 3 comprend un ratio agar : κ-carraghénanes 50 : 50.

### DESCRIPTION DETAILLEE DE L'INVENTION

Certains termes et expressions utilisés dans le cadre de l'invention sont détaillés ci-après.

Un premier objet de l'invention concerne un milieu de culture pour la détection de microorganismes comprenant
- de l'agar ou de l'agarose
- du κ-carraghénane

Le milieu de culture selon l'invention comprend en outre une peptone en tant que source d'acides aminés et d'azote.

Par «milieu de culture», on entend un milieu comprenant tous les éléments nécessaires à la croissance de micro-organismes. D'une façon générale, le milieu de culture gélosé comprend:
- une source de carbone apportée généralement par des sucres
- une source d'acides aminés et d'azote apportée par des peptones
- différents sels et tampon
- de l'eau déminéralisée
- un agent gélifiant

D'autres ingrédients peuvent être ajoutés, comme par exemple des agents sélectifs. Le milieu peut comprendre également un colorant.

Selon la présente invention, le milieu de culture peut se présenter sous forme de gel prêt à l'emploi c'est-à-dire prêt à l'ensemencement dans une boite de Pétri. On parle également de milieu de culture gélifié, ou encore de milieu sous forme solide. Le milieu de culture peut également être sous forme de poudre. Dans ce cas, il est ensuite réhydraté, chauffé/stérilisé puis coulé dans une boite de Pétri afin de former un milieu gélifié prêt à l'emploi. L'agar est l'agent gélifiant traditionnel utilisé en microbiologie pour la culture des micro-organismes, mais il est possible d'utiliser en outre d'autres agents gélifiants comme par exemple la gomme gellane, la pectine, la gomme de guar, la gélatine, l'agarose ainsi que d'autres gélifiants naturels ou artificiels.

L'agar est un polysaccharide non ramifié obtenu à partir des parois cellulaires de certaines espèces d'algues rouges, principalement de *tengusa* (*Gelidiaceae*) et *d'ogonori* (*Gracilaria*)*.* L'agar permet d'avoir un réseau suffisamment stable et propice à la prolifération bactérienne sans pour autant servir de nourriture à celles-ci, préservant la structure matricielle du gélifiant. Un certain nombre de préparations sont disponibles dans le commerce, comme par exemple l'agar Columbia, la gélose Trypcase-soja, la gélose Mac Conkey, la gélose Mueller Hinton ou plus généralement celles décrites dans le Handbook of Microbiological Media.

Selon la présente invention, le milieu de culture peut comprendre de l'agarose qui est un polysaccharide extrait de l'agar, composé de la répétition d'un diholoside. L'agarose est la partie gélifiante de l'agar.

Selon la présente invention, le milieu de culture comprend du κ-carraghénane. Les carraghénanes sont des polysaccharides anioniques extraits d'algues rouges principalement de *Gigarina, Chondrus et Eucheuma.* Ils existent sous plusieurs formes et les principales classes commerciales sont :
- La forme κ-carraghénane, gel rigide en présence d'ions monovalents comme potassium, sodium, rubidium, lithium, etc. ou des ions divalents comme calcium ou magnésium. Il provient essentiellement de l'algue *kappaphycus alvarezii*
- La forme ι-carraghénane, gel mou en présence d'ions calcium. Il provient principalement d'algue *Eucheuma denticulatum.*
- La forme λ-carraghénane qui ne forme pas de gel.

D'une manière tout à fait surprenante, il a été constaté qu'un milieu de culture comprenant un mélange d'agar et κ-carraghénane permettait d'améliorer la résistance à la rétraction du milieu. En effet, les milieux de culture, dans leur utilisation de routine, vont être incubés à des températures entre 25 et 45°C pendant des périodes pouvant aller dans certains cas jusqu'à 28 jours. Pour les applications de contrôle de l'environnement pharmaceutique, les milieux gélosés sont exposés pendant plusieurs heures à des flux d'air laminaires déshydratants. Dans tous ces cas d'utilisations une forte déshydratation des milieux de culture se produit et va conduire à une déstabilisation du réseau d'agar et à l'apparition de tensions à l'intérieur du gel. Pour les milieux de culture avec de l'agar, ce phénomène peut entrainer l'apparition de rétractions, craquelures et /ou décollements. Ces défauts ont un impact direct sur la performance microbiologique des milieux. Les solutions techniques permettant de limiter leur apparition malgré la déshydratation représentent un avantage important dans la qualité des résultats rendus par ces produits. Ainsi une plus grande résistance à la rétraction des géloses permet d'obtenir des péremptions plus longues.

De façon préférentielle la concentration du mélange agar et κ-carraghénanes est comprise entre 10g/l et 30g/l, préférentiellement entre 10 et 20g/l. Encore plus préférentiellement entre 13 et 17 g/l.

Avantageusement, le ratio pondéral d'agar : κ-carraghénane est compris entre 80 : 20 et 30 : 70.

On entend par « rétraction » un décollement partiel ou total du milieu de culture de la paroi latérale de la boite de Pétri. Cela entraine ensuite une diminution de la surface. La résistance à la rétraction peut être mesurée par le « Test de Déshydratation Extrême » qui consiste à exposer les boites sous une hotte à flux d'air laminaire vertical pendant 60 heures sans couvercle. Les milieux de culture vont se déshydrater durant l'exposition. La surface de chaque milieu déshydraté est mesurée ensuite à l'aide d'un instrument de lecture de boîte tel que le Scan^{®} 4000. Un milieu de culture ayant une faible résistance à la rétraction va se décoller rapidement de la paroi latérale de la boîte durant le test et va continuer à se rétrécir jusqu'à obtenir une fraction de la surface initiale totalement sèche. En revanche, un milieu de culture ayant une bonne résistance à la rétraction ne se décollera de la paroi latérale de la boîte que plus tard voire jamais durant le test et la surface du milieu séché diminuera faiblement voir pas du tout.

Le ratio agar : κ-carraghénane a également un effet sur la gélification de la gélose. Ces propriétés sont mesurées par la température de gélification, la viscosité et le module élastique de la gélose.

La température de gélification est mesurée à l'aide d'un rhéomètre. Les échantillons de milieux de culture liquides sont déposés sur la plaque Peltier du rhéomètre préchauffée à une température plus élevée que la température de gélification de l'échantillon. Les échantillons sont ensuite refroidis à 20°C, avec une vitesse de refroidissement de 3°C/min, vitesse similaire à celle en production. Lors du refroidissement, une déformation oscillante est imposée sur l'échantillon qui permet d'étudier les propriétés viscoélastiques et les changements des phases « liquides » à « solides » de l'échantillon. La température de gélification est définie par une augmentation rapide du module élastique ou de la viscosité qui représente le début de la formation des réseaux de gélifiant et d'une solidification du milieu.

La température de gélification peut dépendre également des conditions physico-chimiques du milieu tels que la charge en ions ou encore le pH. Certains milieux comprennent de fortes concentrations en sels (Potassium, Sodium, Calcium) tels que certains milieux sélectifs. Or la présence d'ions augmente la température de gélification. Une température de gélification comprise entre 30 et 45°C est particulièrement intéressante car elle permet une production du milieu facilement industrialisable. En effet, il ne sera pas nécessaire de maintenir le milieu à de haute température pour éviter sa gélification avant d'être coulé dans les boites. De plus, dans le cas d'une préparation du milieu en laboratoire, une température inférieure à 60°C permet d'éviter les brûlures. D'autre part, un tel milieu permet également l'ajout de composés thermosensibles tels que le sang ou des antibiotiques qui ne sont alors pas détériorés par la chaleur.

La viscosité se mesure à l'aide d'un rhéomètre. La viscosité correspond à la résistance d'un fluide au changement de forme. Elle détermine la vitesse de mouvement du fluide. Plus le liquide est visqueux, plus le mouvement est lent. Pour le milieu de culture avec un mix agar : κ-carraghénane, une viscosité similaire à un milieu avec 100% d'agar est particulièrement intéressante car elle permet une production du milieu sans nécessité de modification des paramètres du procédé de fabrication, à savoir la vitesse d'agitation, ou encore la pression de pompe pour la répartition.

Le module élastique est une grandeur intrinsèque d'un matériau, définie par le rapport d'une contrainte à la déformation élastique provoquée par cette contrainte. Pour le milieu de culture, il représente la dureté du milieu. Plus le milieu est mou, plus le module élastique est faible. Un milieu de culture avec un module élastique supérieur à 5 kPa est nécessaire pour que le milieu soit correctement ensemencé manuellement ou automatiquement. Il se mesure à l'aide d'un rhéomètre. Les échantillons de milieux de culture liquides sont déposés sur la plaque Peltier du rhéomètre préchauffée à une température plus élevée que la température de gélification de l'échantillon. Les échantillons sont ensuite refroidies à 20°C, avec une vitesse de refroidissement de 3°C/min, vitesse similaire à celle en production. Lors du refroidissement, une déformation oscillante est imposée sur l'échantillon qui permet d'étudier les propriétés viscoélastiques et les changements des phases « liquides » à « solides » de l'échantillon. Durant le refroidissement, le module élastique augmente avec la gélification du milieu. Il atteint ensuite une valeur stable ce qui signifie la finalisation de la gélification. Cette valeur finale correspond au module élastique du milieu.

Ainsi dans un mode particulier de l'invention, le milieu a une concentration en sels inférieure à 5 g/l et un ratio pondéral d'agar : κ-carraghénane compris entre 80 : 20 et 60 : 40. Les milieux ayant une concentration en sel inférieure à 5g/l, sont bien connu de l'homme du métier. On peut citer par exemple le milieu Sabouraud Dextrose agar. La substitution du gélifiant d'un milieu à faible charge ionique par un mélange d'agar : κ-carraghénane ayant un ratio compris entre 80 : 20 et 60 : 40, permet d'obtenir un milieu ayant un module élastique approprié, soit supérieur à 5 kPa, facilitant son ensemencement. Il permet également d'obtenir une température de gélification entre 30°C et 45°C, et une viscosité similaire à un milieu non modifié avec 100% d'agar, ce qui facilite son industrialisation.

Ce ratio compris entre 80 : 20 et 60 : 40 est également particulièrement approprié pour un milieu avec une grande concentration en sel au-delà de 20 g/l. Ainsi dans un mode particulier de l'invention, le milieu a une concentration en sels supérieure à 20 g/l et le ratio pondéral d'agar : κ-carraghénane est compris entre 80 : 20 et 60 : 40. Les milieux ayant une forte concentration en sels, c'est-à-dire supérieure à 20 g/l, sont bien connu de l'homme du métier. On peut citer par exemple le milieu CHAPMAN. La substitution du gélifiant d'un milieu à forte concentration en sel par un mélange d'agar : κ-carraghénane ayant un ratio compris 80 : 20 et 60 : 40 permet d'avoir un module élastique approprié, soit supérieur à 5 kPa, facilitant son ensemencement. Il a également une température de gélification entre 30°C et 45°C, et une viscosité similaire à un milieu non modifié avec 100% d'agar, facilitant son industrialisation.

Dans un autre mode particulier de l'invention, le milieu selon l'invention a une concentration en sel comprise entre 5g/l et 20 g/l, et un ratio pondéral d'agar : κ-carraghénane compris entre 80 : 20 et 30 : 70. Les milieux ayant une concentration en sels classique c'est-à-dire comprise entre 5-20 g/l, sont bien connus de l'homme du métier. On peut citer par exemple le milieu TSA. La substitution du gélifiant d'un milieu ayant une concentration en sels comprise entre 5-20 g/l par un mélange d'agar : κ-carraghénane ayant un ratio compris 80 : 20 - 30 70, permet d'obtenir un milieu avec un module élastique compatible à l'ensemencement, soit supérieur à 5 kPa. Ce ratio permet également d'obtenir une température de gélification entre 30°C et 45°C, une viscosité similaire à un milieu non modifié avec 100% d'agar, ce qui facilite son industrialisation.

Dans un mode de réalisation préféré, le milieu de culture comprend en outre un composé thermosensible. Le composé thermosensible peut être un antibiotique et/ou du sang. Ainsi un milieu de culture selon l'invention avec une température de gélification comprise entre 30°C et 45°C permet la conservation des composés thermosensibles.

Un autre objet de l'invention concerne un procédé d'obtention d'un milieu caractérisé en ce qu'il consiste essentiellement à:
- mettre en surfusion les composés du milieu de culture afin de les mélanger
- refroidir à une première température au-dessus de la température de gélification du milieu de culture
- Remplir les boites de Pétri avec ce milieu de culture en solution
- laisser refroidir pour atteindre la température de gélification et permettre la formation d'un milieu de culture gélifié, la température de gélification étant comprise entre 30°C et 45°C. D'une façon générale, les composés du milieu sont mis en surfusion à une température supérieure à 80°C, voir supérieure à 100°C pour permettre leur stérilisation.

Les composés sont alors mélangés. Le milieu de culture en surfusion est ensuite refroidi à une température qui reste au-dessus de la température de gélification du milieu de culture. En général, la température est entre 50 et 60°C.

Si des composés thermosensibles sont nécessaires, ils sont alors ajoutés.

Le milieu de culture est ensuite coulé dans les boites de Pétri. Il atteint alors sa température de gélification qui est la température qui caractérise la transition de phase de « liquide » à « solide ».

Un autre objet de l'invention concerne un milieu de culture comprenant un mélange d'agar : κ-carraghénane ayant un ratio pondéral compris entre 80 : 20 et 30 : 70 et une température de gélification comprise entre 30°C et 45°C.

Ce milieu constitue alors un produit intermédiaire dans le procédé d'obtention du milieu lorsque sa température atteint la température de transition de phase de « liquide » à « solide ».

Un autre objet de l'invention concerne un milieu selon l'invention gélifié dans une boite de Pétri.

Un autre objet de l'invention concerne une méthode de culture microbiologique in vitro, dans laquelle des microorganismes susceptibles d'être présents dans un échantillon sont ensemencés dans ou sur un milieu de culture selon l'invention.

Un autre objet de l'invention concerne une méthode de détection d'un micro-organisme cible dans un échantillon susceptible de le contenir comprenant les étapes suivantes :
- mettre en contact ledit échantillon avec un milieu de culture gélifié selon l'invention
- incuber
- détecter la présence dudit microorganisme.

La présente invention est illustrée de manière non limitative à partir des exemples suivants

### Exemples

### Exemple 1 : Préparation de milieux de culture selon l'invention et autres milieux de culture

Différentes concentrations en gélifiants (agar, carraghénane) ont été utilisées : de 0 à 15 g/l. Différents ratios pondéraux agar : carraghénane ont été utilisés pour constituer le mix gélifiant agar : carraghénane :
- 0 : 100 ; 30 : 70 ; 40 : 60 ; 50 : 50 ; 70 : 30 ; 20 : 80 ; 100 : 0.

Différents milieux, appelés milieux modifiés, ont été préparés à partir d'une base de milieu TSA, CHAPMAN ou Sabouraud Dextrose Agar (tableaux ci-dessous) dans lequel l'agar a été remplacé par un mix agar : carraghénane dont les ratios sont mentionnés dans les exemples.

**[Table 1] Formule du milieu TSA modifié**

| | |
|---|---|
| peptone de caséine (bovine) | 15g |
| Peptone de soja | 5g |
| Extrait de levure | 6g |
| Chlorure de Sodium | 5g |
| Pyruvate de Sodium | 2g |
| Lécithine de soja | 0.7g |
| Polysorbate 80 | 5g |
| Thiosulfate de sodium 5H2O | 0.05g |
| L-histidine | 1g |
| Gélifiant | 15g |
| Eau purifiée | 1L |
| pH 7.3 | |

**[Table 2] Formule du milieu CHAPMAN modifié**

| | |
|---|---|
| Extrait de viande (bovine ou porcine) | 1g |
| Peptone de caséine (bovine ou porcine) | 5g |
| Peptone de viande (bovine ou porcine) | 5g |
| Chlorure de sodium | 75g |
| D mannitol | 10g |
| Gélifiant | 15g |
| Rouge phénol | 25mg |
| Eau purifiée | 1L |
| pH 7.4 | |

**[Table 3] Formule du milieu Sabouraud Dextrose Agar modifié**

| | |
|---|---|
| Dextrose | 15g |
| Digestion pancréatique de caséine (bovin) | 5g |
| Digestion pancréatique de tissu animal (bovin ou porcin) | 5g |
| Gélifiant | 15g |
| Eau purifiée | 1L |
| pH 5,6 | |

La préparation suit les étapes suivantes :
1) Les agars, les carraghénanes et les autres matières premières sont pesés selon la formule de chaque milieu de culture.
2) L'ensemble des matières premières est dilué avec de l'eau déminéralisée.
3) L'ensemble est mis sous agitation et chauffé jusqu'à ébullition afin de dissoudre complètement les matières premiers.
4) La solution est autoclavée en cycle liquide, avec un plateau à 120°C durant 16 min. Les étapes 3) et 4) peuvent être également réalisées avec un instrument automatisé de préparation de milieux de culture type Masterclave^{®}.
5) 20-30 ml de milieu sont versés à chaud c'est-à-dire à une température supérieure à la température de gélification du milieu dans les boîtes de Pétri 90mm sans couvercle.
6) les couvercles des boîtes sont remis.
7) Les boîtes de milieux de culture sont stockées à 2-8°C.

### Exemple 2 : Test de résistance à la rétraction de milieux selon l'invention comprenant de l'agar de fournisseurs différents

Différents types d'agar ont été testés pour l'évaluation de la résistance à la rétraction :
- Agar Européen (Roko; ref 03904182, Lot 180100340)
- Agar Européen (Setexam; ref 03904185, Lot M2019)
- Agar Américan (Roko; ref 3904170, Lot 200201654)
- Agar commercial (Sigma Aldrich, ref 05040 CAS: 9002-18-0, Lot BCCF9819)
- Agar Gracilaria (Roko, ref Rokoagar RGM LAB, Lot 200201758)
- Agarose (Sigma Aldrich, ref A4718 CAS: 9012-36-6, Lot SLCK4183)

Les milieux testés comprenaient soit de l'agar seul soit en mix avec du κ-carraghénane du fournisseur Setexam (Setexam, Reference Danish agar: 03904187, Lot C19152).

Pour les milieux avec agar seul, la concentration de l'agar est à 15g/l.

Pour les milieux avec mix agar : κ-carraghénane, un ratio 50 : 50 a été utilisé. la concentration de gélifiant total est à 15g/l.

La formule TSA a été utilisée pour ce test. Les milieux de culture ont été préparés à l'aide d'un Masterclave^{®}. 30ml du milieu a été ajouté dans chaque boîte de Pétri de 90mm.

La méthode de Test de Déshydratation Extrême a été utilisée pour évaluer la résistance à la rétraction de chaque milieu en boîte de Pétri:
- Trente boîtes de chaque formule ont été exposées sous une hotte à flux d'air laminaire vertical (V=0,45m/s) pendant 60 heures sans couvercle.
- Le milieu de culture est complètement déshydraté après l'exposition avec une perte d'eau totale.
- L'aspect de chaque milieu de culture a été observé post-exposition.
- La surface de chaque milieu de culture séché a été mesurée à l'aide d'un instrument lecteur automatique de boîte Scan^{®} 4000 (réf 438000, fournisseur Interscience).

Les résultats ont été présentés en pourcentage, calculé par rapport à la surface initiale du milieu de culture.

**[Table 4] Résultats de la méthode de Test de Déshydratation Extrême du TSA avec mix agar : κ-carraghénane comprenant différents agars.**

| | **surface milieu séché/surface milieu initial %** | |
|---|---|---|
| | <a> | <b> |
| | Agar : κ-carraghénane = 100: 0 | Agar : κ-carraghénane = 50 : 50 |
| 1 Agarose -Sigma Aldrich | 25,9 | 82,8 |
| 2 Agar -Sigma Aldrich | 42,9 | 88,1 |
| 3 Agar American -Roko | 46,1 | 87,1 |
| 4 Agar Européen -Roko | 46,5 | 91,9 |
| 5 Agar Européen -Setexam | 48,9 | 89,1 |
| 6 Agar Gracilaria -Roko | 60,0 | 90,6 |

**Conclusion** : Comme on peut le constater **figure 1****,** tous les milieux avec 100% d'agar (milieux 1a, 2a, 3a, 4a, 5a, 6a) présentent de très mauvaises résistances à la rétraction. Toutes les géloses se décollent de la paroi de la boîte et rétrécissent au cours du test. L'utilisation d'un mix agar : κ-carraghénane (50 : 50) améliore considérablement la résistance à la rétraction (milieux 1b, 2b, 3b, 4b, 5b, 6b).

D'autre part, il a été observé que le nombre de milieux de culture intacts post-exposition augmente. Les résultats sont présentés à la **figure 1****.** Par exemple, pour le mix agar Européen-Roko : κ-carraghénane (milieu 4b), 43% des milieux de cultures ne présentent pas de rétraction après le Test de Déshydratation Extrême. La surface du milieu de culture séché est d'ailleurs augmentée de 46,5 % à 91,9% comme l'indique le tableau 4.

Ainsi, l'amélioration de la résistance à la rétraction a été observée sur le mix agar : κ-carraghénane avec tous les types d'agar et d'agarose testés dans cette étude, comme montré **figure 1, figure 2** **et tableau 4.**

### Exemple 3 : Test de résistance à la rétraction de milieux comprenant différents carraghénanes (kappa, lambda, iota) et de milieux de cultures selon l'invention

Différents types de carraghénane ont été testés pour l'évaluation de la résistance à la rétraction :
- κ-carraghénane (Setexam, Reference Danish agar: 03904187, Lot C19152)
- κ-carraghénane (Roko, Reference Rokogel 4600: MV210120, Lot 210200659)
- κ-carraghénane (Sigma Aldrich, ref 22048, Lot BCCF0613)
- ι-carraghénane (Sigma Aldrich ref C1138, Lot SLCG0678)
- λ-carraghénane (Sigma Aldrich, ref 22049, Lot BCBP8978V)

Chaque carraghénane a été testé en mix avec l'agar européen (Setexam, ref. 03904185, Lot M2019). Un ratio agar : carraghénane = 50 : 50 a été utilisé. La formule TSA a été utilisée pour ce test. Les milieux de culture ont été préparés à l'aide d'un Masterclave^{®}. 30ml du milieu a été ajouté dans chaque boîte de Pétri 90mm.

La méthode de Test de Déshydratation Extrême telle que décrite à l'exemple 2 a été utilisée pour évaluer la résistance à la rétraction de chaque milieu en boîte de Pétri. Les résultats ont été présentés en pourcentage calculé par rapport à la surface initiale du milieu de culture.

**[Table 5] Résultats de la méthode de Test de Déshydratation Extrême du TSA avec mix agar : carraghénane comprenant différents carraghénanes.**

| | | **Surface milieu séché/surface initiale %** |
|---|---|---|
| **Agar: Carraghénane** = **100 :0** | 1 - Agar Européen | 46,5 |
| **Aga**r **: Carraghénane =50 :50** | 2- κ-carraghénane -Sigma Aldrich | 80,0 |
| | 3- κ-carraghénane -Setexam | 91,4 |
| | 4- κ-carraghénane -Roko | 94,8 |
| | 5- λ-carraghénane -Sigma Aldrich | 54,0 |
| | 6 ι- carraghénane -Sigma Aldrich | 54,2 |

Comme on peut le constater **tableau 5** et **figure 3****,** l'utilisation de mix agar : κ-carraghénane améliore la résistance à la rétraction (milieux 2, 3, 4). Cette amélioration a été observée pour les trois κ-carraghénanes testées provenant de différents fournisseurs. Par exemple, pour le mix agar : κ-carraghénane (Setexam), la surface du milieu de culture post-exposition est d'environ 91% de la taille initiale au lieu de 46 % pour le milieu avec agar. Pour le mix agar : κ-carraghénane (Sigma Aldrich) et mix agar : κ-carraghénane (Roko), les surfaces des géloses post-exposition sont également améliorées.

Cependant, le mix avec λ-carraghénane (milieu 5) ou i-carraghénane (milieu 6) n'améliore pas la résistance à la rétraction du milieu. Au cours du temps, tous les milieux rétrécissent et deviennent un petit film séché avec seulement 50 % de surface résiduelle par rapport à leur surface initiale.

En conclusion, l'amélioration de la résistance à la rétraction a été observée sur les mix avec toutes les κ-carraghénanes testées dans cette étude. En revanche, le λ-carraghénane et le λ-carraghénane n'améliorent pas les performances liées à la rétraction.

### Exemple 4 : Test de résistance à la rétraction des milieux de cultures selon l'invention avec différents ratios agar : κ-carraghénane

Une série de ratio agar : κ-carraghénane a été testée pour l'évaluation de la résistance à la rétraction (100 : 0, 70 : 30, 60 : 40, 50 : 50, 30 : 70, 0 : 100) avec une concentration totale du gélifiant égale à 15g/l. L'agar Européen (Setexam, ref 03904185, Lot M2019) et κ-carraghénane (Setexam, ref 03904187, Lot C19152) ont été utilisés. La formule TSA a été utilisée pour ce test. Les milieux de culture ont été préparés à l'aide d'un Masterclave^{®}. 30ml du milieu ont été ajoutés dans chaque boîte de Petri 90mm.

La méthode de Test de Déshydratation Extrême telle que décrite à l'exemple 2 a été utilisée pour évaluer la résistance à la rétraction de ces milieux.

**[Table 6] Résultats de la méthode de Test de Déshydratation Extrême du TSA avec mix agar : κ-carraghénane (ratio 100 : 0, 70 : 30, 60 : 40, 50 : 50, 30 : 70 et 0 : 100)**

| | Agar : κ-carraghénane | **Surface séchée** / **surface initiale %** |
|---|---|---|
| Milieu 1 | 100 : 0 | 46,5 |
| Milieu 2 | 70 : 30 | 73,9 |
| Milieu 3 | 60 : 40 | 89,4 |
| Milieu 4 | 50 : 50 | 91,8 |
| Milieu 5 | 30 : 70 | 97,4 |
| Milieu 6 | 0 : 100 | 90,5 |

Le milieu avec 100% d'agar présente une mauvaise résistance à la rétraction. Tous les milieux se décollent de la paroi de la boîte et rétrécissent au cours du test. La surface du milieu séché n'est que de 46,5% par rapport à sa surface initiale.

L'utilisation d'un mix agar : κ-carraghénane améliore considérablement la résistance à la rétraction par rapport au milieu à la gélose avec 100% d'agar.

Avec un ratio agar : κ-carraghénane de 70:30, tous les milieux rétractent au cours du test. Cependant, la surface moyenne du milieu séché est de 73,9% par rapport à la surface initiale, au lieu de 46,5% pour la TSA avec 100% d'agar comme l'indique le tableau 6.

Avec un ratio agar : κ-carraghénane de 50 : 50 (milieu 4), 43% des milieux ne présentent pas de rétraction post Test de Déshydratation Extrême, comme montré **figure 4****.** La surface moyenne du milieu séché est de 91,8% par rapport à la surface initiale comme l'indique le **tableau 6.**

Avec un ratio agar : κ-carraghénane de 30 : 70 (milieu 5), 70% des milieux ne présentent pas de rétraction post Test de Déshydratation Extrême comme montré **figure 4****.** La surface moyenne du milieu séché est augmentée à 97,4% par rapport à la surface initiale comme l'indique le **tableau 6.**

**Conclusion** : l'utilisation du mix agar : κ-carraghénane permet une amélioration significative de la résistance à rétraction du milieu. Cette amélioration a été observée sur différents ratios agar : κ-carraghénane : 70 : 30 ; 60 : 40 ; 50 : 50 ; 30 : 70.

### Exemple 5: Test de gélification des milieux de cultures selon l'invention avec différents ratios agar :κ-carraghénane

Une série de ratio agar : κ-carraghénane a été testée (100 : 0, 80 : 20, 70 : 30, 60 : 40, 50 : 50, 30 : 70, 0 : 100) avec une concentration totale du gélifiant égale à 15g/l. L'agar Européen (Setexam) et κ-carraghénane (Setexam ou Roko) ont été utilisés avec les formules TSA, CHAPMAN, et Sabouraud Dextrose Agar. Les solutions ont été autoclavées en cycle liquide, avec un plateau à 120°C durant 16 min puis mises dans un bain-marie préchauffé à 80°C. La température de la gélification de chaque milieu a été mesurée à l'aide d'un rhéomètre Discovery HR-2 (TA Instruments).

Les résultats obtenus avec le milieu TSA modifié sont représentés **figure 5 (a) et 5 (b)****.**

Pour le milieu TSA avec mix agar : κ-carraghénane, les formules avec ratio 70 : 30, 50 : 50 et 30 : 70 possèdent une température de gélification entre 30°C et 45°C. Si on augmente la part de κ-carraghénane de plus de 70% (milieu 5), la température de gélification dépasse 45°C comme montré à la **figure 5 (a)****.** La viscosité du milieu devient également très importante, comme montré à la **figure 5 (b)****.**

Une augmentation de la part de κ-carraghénane au-delà de 70% du mix peut rendre l'industrialisation difficile. Les modifications de procédé de production sont nécessaires. De plus la fabrication des milieux avec additifs thermosensibles (antibiotiques) ou des milieux au sang (géloses au sang de mouton ou de cheval) devient également impossible.

**Conclusion** : Pour le milieu TSA modifié ou les milieux de culture avec des concentrations en sels comprises entre 5-20g/l, il est préférable d'avoir une part en κ-carraghénane égale ou inférieure à 70% dans le mix agar : κ-carraghénane.

Les résultats obtenus avec le milieu **CHAPMAN modifié** sont représentés **figure 6****.**

La température de gélification pour un milieu CHAPMAN avec 100% d'agar est à environ 40°C (milieu 1). Pour le mix agar : κ-carraghénane, les milieux avec ratio 80 : 20 à 60 : 40 (milieux 2, 3, 4) possèdent une température de gélification similaire à celle du milieu CHAPMAN.

Les milieux avec ratio 50 : 50 ou avec une part en κ-carraghénane supérieur à 50% (milieux 5 et 6) ont une température de gélification très importante (>60°C). Ceci peut rendre l'industrialisation difficile. La fabrication des milieux avec additifs thermosensibles (antibiotiques) ou des milieux au sang (géloses au sang de mouton ou de cheval) devient également impossible.

**Conclusion** : Pour le milieu CHAPMAN ou les autres milieux de culture avec fortes charges en ions comprenant une concentration en sels supérieure à 20g/l, l'utilisation d'un ratio 80 : 20 à 60 : 40 est préférée. La présence des ions accélère la gélification de κ-carraghénane et augmente la température de gélification.

Les résultats obtenus sur le milieu Sabouraud Dextrose Agar **(SDA)** sont représentés **figure** 7.

La température de gélification (figure 7a) pour un milieu SDA avec 100% d'agar est à environ 34°C (milieu 1). Pour le mix agar : κ-carraghénane, les milieux avec ratio 80 :20 à 50 :50 (milieu 2 à milieu 5) possèdent une température de gélification entre 30°C - 45°C. Les milieux avec ratio 30 : 70 ou avec une part en κ-carraghénane supérieure à 70% (milieu 6 et 7) ont une température de gélification très faible (<30°C) comme indique **figure 7(a)****.**

Le module élastique du milieu (figure 7b) est également mesuré par le rhéomètre. Pour le mix agar : κ-carraghénane, les milieux avec ratio 80 : 20 à 60 : 40 (milieux 2, 3, 4) présentent un module élastique acceptable (> 5 kPa). Les milieux avec ratio 50 : 50 ou avec une part en κ-carraghénane supérieure à 50 (milieux 5, 6, 7) sont très mous. Les milieux ne sont pas assez solidifiés pour être manipulés ou ensemencés.

**Conclusion** : Pour le milieu SDA ou les autres milieux de culture avec faibles charges en ions ayant une concentration en sel inférieure à 5g/l, l'utilisation d'un ratio 80 : 20 à 60 : 40 est préférée.

### Exemple 6 : Détection de microorganismes à l'aide de milieux de cultures selon l'invention

Afin d'évaluer les performances microbiologiques des milieux, les tests de la croissance de microorganismes sont réalisés avec les souches répertoriées dans le tableau ci-dessous. Les souches utilisées proviennent du BioBall^{®} MultiShot 550.

Deux boîtes de chaque milieu ont été inoculées pour chaque micro-organisme et ont été incubées comme spécifié dans le tableau ci-dessous. Après l'incubation, les boîtes ont été dénombrées à l'aide d'un instrument de lecture automatique de boîte Scan^{®} 4000 (réf 438000, fournisseur Interscience).

Le taux de récupération (TR) a été calculé. Il correspond au ratio du nombre de colonies sur boîtes testées sur le nombre de colonies sur boîtes témoins avec un milieu de culture non modifié.

Le taux de récupération souhaité est de 50 à 200% comme conseillé par les Pharmacopées.

La formule TSA et la formule CHAPMAN ont été utilisées pour ce test.

**[Table 7] Micro-organisme testé pour milieu TSA :**

| Micro-organisme | ATCC | Source Inoculum | Inoculum | Temps d' incubation | Température d'incubation |
|---|---|---|---|---|---|
| *Staphylococcus aureus* | ATCC 6538 | BioBall Multishot | BioBall: | 24h ± 4h | 30-35°C |
| *Pseudomonas aeruginosa* | ATCC 9027 | | | | |
| *Escherichia coli* | ATCC 8739 | | | | |
| *Bacillus subtilis* | ATCC 6633 | 550 CFU | 100 µl | | |
| *Candida albicans* | ATCC 10231 | | | 48h ± 6h | |
| *Aspergillus brasiliensis* | ATCC 16404 | | | | |

**[Table 8] Micro-organisme testé pour milieu CHAPMAN :**

| **Micro-organisme** | **ATCC** | **Source Inoculum** | **Inoculum** | **Temps d'incubation** | **Temperature d'incubation** |
|---|---|---|---|---|---|
| *Staphylococcus aureus* | ATCC 6538 | BioBall Multishot | BioBall: | 24h-72h | 30-35°C |
| | | 550 CFU | 100 µl | | |

Les résultats sont indiqués dans le tableau ci-dessous.

**[Table 9] Taux de recouvrement du TSA avec mix agar : κ-carraghénane (ratio 70 : 30, 50 : 50, et 30 : 70) par rapport aux boîtes témoins avec de un milieu TSA non modifié.**

| **Micro-organisme** | **70:30** | **50:50** | **30:70** |
|---|---|---|---|
| *Staphylococcus aureus* | 87% | 112% | 79% |
| *Bacillus subtilis* | 91% | 93% | 82% |
| *Escherichia coli* | 86% | 104% | 69% |
| *Pseudomonas aeruginosa* | 90% | 86% | 90% |
| *Candida albicans* | 75% | 83% | 88% |
| *Aspergillus brasiliensis* | 121% | 95% | 121% |

**[Table 10] Taux de recouvrement du CHAPMAN avec mix agar : κ-carraghénane (ratio 80 : 20, 50 : 50, 60 : 40 ) par rapport à milieu CHAPMAN non modifié.**

| **Micro-organisme** | **80:20** | **50:50** | **60:40** |
|---|---|---|---|
| *Staphylococcus aureus* | 106% | 107% | 89% |

**Conclusion** : Les milieux avec mix agar : κ-carraghénane présentent de bonne détection et de bonne croissance des micro-organismes. Les taux de récupération sont similaires au milieu non modifié, et bien conformes à la demande de Pharmacopée, soit compris entre 50%-200% pour tous les micro-organismes testés. Les tailles et les morphologies des colonies des boites 1b à boite 6b (figure 8) ensemencées respectivement par les microorganismes mentionnés tableau 9 sur un milieu TSA avec un ratio agar : κ-carraghénane 70 : 30, sont également très similaires avec celles des boîtes témoins (boites la à boite 6a) comprenant un milieu TSA avec 100% d'agar. De même pour les colonies de *Staphylococcus aureus* **(****figure 9****)** ensemencées sur un milieu CHAPMAN avec un mix agar : κ-carraghénane 80 : 20 (boite 2) et 50 : 50 (boite 3) versus un milieu CHAPMAN avec 100% d'agar (boite 1).

### REFERENCES BIBLIOGRAPHIQUES

Lines, "value of the K+ salt of carrageenan as an agar substitute in Routine bacteriological media", Applied and Environmental Microbiology, Dec 1977, p 637-639

## Revendications

1. Milieu de culture pour la détection de microorganismes comprenant
- de l'agar ou de l'agarose
- du κ-carraghénane

2. Milieu de culture selon la revendication 1 comprenant en outre des peptones.

3. Milieu de culture selon la revendication 1 ou 2 **caractérisé en ce que** la concentration du mélange agar et κ-carraghénane est compris entre 10g/l et 30g/l, préférentiellement entre 10g/l et 20g/l.

4. Milieu de culture selon l'une quelconque des revendications précédentes **caractérisé en ce que** le ratio pondéral d'agar : κ-carraghénane est compris entre 80 : 20 et 30 : 70.

5. Milieu de culture selon l'une quelconque des revendications précédentes **caractérisé en ce que** la concentration en sels présents dans le milieu est inférieure à 5 g/l et le ratio pondéral d'agar : κ-carraghénane est compris entre 80 : 20 et 60 : 40.

6. Milieu de culture selon l'une quelconque des revendications précédentes **caractérisé en ce que** la concentration en sels présents dans le milieu est comprise entre 5g/l et 20 g/l et le ratio pondéral d'agar : κ-carraghénane est compris entre 80 : 20 et 30 : 70.

7. Milieu de culture selon l'une quelconque des revendications précédentes **caractérisé en ce que** la concentration en sels présents dans le milieu est supérieure à 20 g/l et le ratio pondéral d'agar : κ-carraghénane est compris entre 80 : 20 et 60 : 40.

8. Milieu de culture selon l'une quelconque des revendications précédentes caractérisé en ce ledit milieu de culture comprend en outre un composé thermosensible.

9. Milieu de culture selon la revendication 8 caractérisé en ce le composé thermosensible est un antibiotique.

10. Milieu de culture selon l'une quelconque des revendications 8 ou 9 caractérisé en ce le composé thermosensible est du sang.

11. Milieu de culture selon l'une quelconque des revendications 1 à 10 **caractérisé en ce qu'**il est gélifié dans une boite de Pétri.

12. Procédé d'obtention d'un milieu de culture selon l'une quelconque des revendications 1 à 11 **caractérisé en ce qu'**il consiste essentiellement à:
- mettre en surfusion les composés du milieu de culture afin de les mélanger
- refroidir à une première température au-dessus de la température de gélification du milieu de culture
- remplir les boites de Pétri avec ce milieu de culture en solution
- laisser refroidir pour atteindre la température de gélification et permettre la formation d'un milieu de culture gélifié, la température de gélification étant comprise entre 30°C et 45°C.

13. Milieu de culture comprenant un mélange d'agar : κ-carraghénane ayant un ratio pondéral compris entre 80 : 20 et 30 : 70 et une température de gélification comprise entre 30°C et 45°C.

14. Méthode de culture microbiologique *in vitro,* dans laquelle des microorganismes susceptibles d'être présents dans un échantillon sont ensemencés dans ou sur un milieu de culture dans une boite de Pétri tel que décrit à l'une quelconque des revendications 1 à 11.

15. Méthode de détection d'un micro-organisme cible dans un échantillon susceptible de le contenir comprenant les étapes suivantes :
- mettre en contact ledit échantillon avec un milieu de culture gélifié selon l'une quelconque des revendications 1 à 11
- incuber
- détecter la présence dudit microorganisme.
